# EUROPEAN PATENT APPLICATION

(11) **EP 2 946 663 A1**
(43) Date of publication of application: **25.11.2015**
(21) Application number: 14169619.5
(22) Date of filing: 23.05.2014
(51) Int. Cl.: A01K 67/027, C07K 14/705, C12N 15/85

(54) **Genetically engineered murine animal as a model for Primary Membraneous Nephropathy**

(71) Applicant: Universitätsklinikum Hamburg-Eppendorf, 20246 Hamburg (DE)
(72) Inventor: Stahl, Rolf, 22359 Hamburg (DE); Zahner, Gunther, 25421 Pinneberg (DE)
(74) Representative: Stüven, Ralf

(57) **Abstract**

The invention relates to a genetically engineered murine animal, in particular a gene knockin mouse, as animal model for human disease. The technical problem to be solved is to provide a suitable means for assisting research for the causes and the development of and possible therapies and/or therapeutic agents for primary membranous nephropathy. For this purpose, the invention provides, in a first aspect, a genetically engineered murine animal, having expressibly integrated into the genome of all its germ cells and somatic cells a nucleic acid sequence coding for a fusion protein comprising the extracellular domains of the human phospholipase A2 receptor (hPLA2R), an ER targeting signal peptide and a membrane anchoring domain.

## Description

### {Technical Field}

The invention relates to a genetically engineered murine animal, in particular a gene knockin mouse, as animal model for human disease.

### {Background}

In human kidneys, the phospholipase A2 receptor (PLA2R) is expressed on podocytes and probably the major auto-antigen in primary membranous nephropathy (MN, also called membranous glomerulonephritis, MGN) [1]. Binding of circulating autoantibodies to the receptor leads to the formation of immune complexes and initiates podocyte injury and proteinuria. Even though sufficient indirect evidence suggests that this pathomechanism is involved in primary MN, the ultimate experimental proof has yet to be provided. There is still a need for a means enabling further insight into the causes and the development of this disease, and for testing possible therapies or therapeutic agents.

### {Summary of Invention}

The technical problem to be solved is to provide a suitable means for assisting research for the causes and the development of and possible therapies and/or therapeutic agents for primary membranous nephropathy.

In order to solve this problem the invention provides, in a first aspect, a genetically engineered murine animal, having expressibly integrated into the genome of all its germ cells and somatic cells a nucleic acid sequence coding for a fusion protein comprising the extracellular domains of the human phospholipase A2 receptor hPLA2R, an ER targeting signal peptide and a membrane anchoring domain.

In a second aspect the invention provides a genetically engineered murine animal, obtainable by transfecting an embryonic stem cell of a murine animal with a vector comprising a nucleic acid encoding a fusion protein comprising the extracellular domains of hPLA2R, an ER targeting signal peptide and a membrane anchoring domain, integrating the transfected embryonic stem cell into a blastocyst of a non-transfected murine animal, crossing the chimeric animal resulting from the blastocyst with a non-transfected murine animal and selecting a transfected murine animal from the offspring.

In a third aspect the invention provides a genetically engineered murine animal, expressing exclusively in its podocytes a fusion protein comprising the extracellular domains of the human phospholipase A2 receptor hPLA2R, an ER targeting signal peptide and a membrane anchoring domain.

In a fourth aspect the invention provides a process for manufacturing a genetically engineered murine animal, the process comprising the steps of transfecting an embryonic stem cell of a murine animal, preferably a mouse or rat, further preferred a mouse, with a vector comprising a nucleic acid encoding a fusion protein comprising the extracellular domains of the human phospholipase A2 receptor hPLA2R, an ER targeting signal peptide and a membrane anchoring domain, integrating the embryonic stem cell into a blastocyst of a non-transfected murine animal, crossing the chimeric animal resulting from the blastocyst with a non-transfected murine animal and selecting a transfected murine animal from the offspring.

The invention provides murine animal models for e.g. research and drug testing regarding MN, i.e. genetically modified murine animals, e.g. mice, that express or can be made to express the human PLA2R. Since rodents normally do not express PLA2R on glomerular podocytes, it was previously impossible to investigate concepts for treating primary membranous nephropathy in wild-type rats or mice. Existing mouse models of MN are often unsatisfactory [7]. The animal models of the current invention are useful not only for answering the principal question of whether PLA2R can be a target in the initiation of MN, but also as a very useful tool for analyzing the formation and resolution of immune complexes and the humoral and cellular mediation of this disease. These models will allow researchers to characterize in more detail the pathogenesis of membranous nephropathy and will help to develop more specific therapies.

### {Brief Description of Drawings}

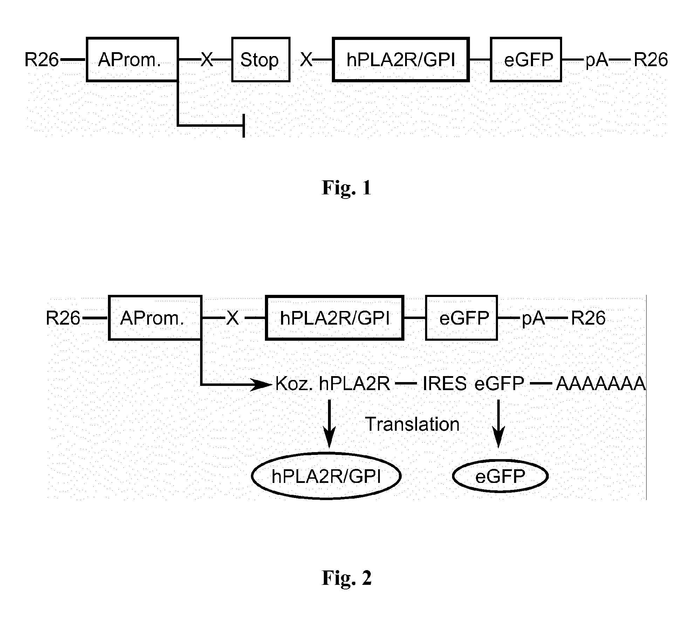

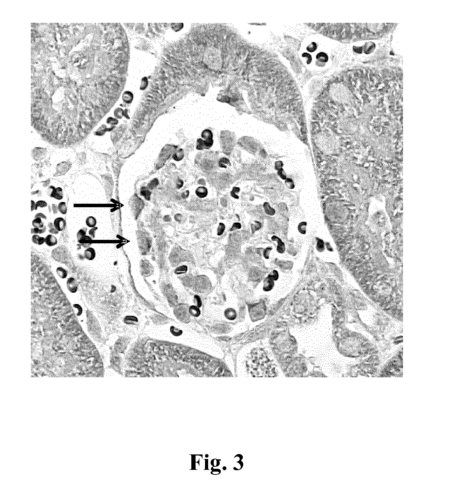

**{****Fig. 1****}**
   Construction of the target vector used to produce hPLA2R/GPI mice. R26 = Rosa26 locus, AProm = chicken actin promoter, eGFP = enhanced green fluorescent protein, pA = poly(A) tail
**{****Fig. 2****}**
   Podocyte-specific activation of gene expression after breeding with Podo-Cre mice. R26 = Rosa26 locus, AProm = chicken actin promoter, eGFP = enhanced green fluorescent protein, pA, AAAAAAA = poly(A) tail, Koz. = Kozak sequence, IRES = internal ribosome entry site
**{****Fig. 3****}**
   Glomerular human PLA2R expression in Podo-Cre hPLA2R/GPI mice. Arrows: PLA2R staining in cell-membranes and cytoplasm of podocytes.

### Description of Embodiments

The current invention provides a novel and realistic animal model for Primary Membraneous Nephropathy (MN) in humans.

In a first aspect the invention relates to a genetically engineered murine animal, having expressibly integrated into the genome of all its germ cells and somatic cells a nucleic acid sequence coding for a fusion protein comprising the extracellular domains of the human phospholipase A2 receptor (hPLA2R), an ER targeting signal peptide and a membrane anchoring domain.

The nucleic acid sequence encoding the fusion protein is stably and expressibly integrated into the genome of the murine animal of the invention, "stably" meaning that the nucleic acid remains a part of the genome of the host animal and is inheritable in a Mendelian fashion, "expressibly" meaning that the nucleic acid is conditionally expressed, i.e. is functionally linked to a promoter driving transcription of the nucleic acid in a living cell, but is only expressed under specific circumstances, e.g. when expression is switched on or enabled by a specific measure, e.g. the activation of a recombinase in the cell. An example is a nucleic acid the expression of which is prevented by a stop cassette downstream of the promoter, e.g. a floxed neomycin stop cassette, the removal of which by e.g. Cre recombinase enables expression (see. e.g. [8]).

Thus, the genetically engineered murine animal of the present invention is prepared to express the human phospholipase A2 receptor (hPLA2R), because it stably contains a nucleic acid coding for it in its genome in expressible form, but it essentially does not express the human phospholipase A2 receptor (hPLA2R) in any of its cells unless the expression is deliberately initiated or enabled.

The phospholipase A2 receptor (PLA2R) is a 180 kDa type I transmembrane glycoprotein, binding several types of phospholipase A2 (sequence see NCBI NM_007366.4). In humans, it is mainly expressed in podocytes of kidney glomerula. The sequence of the extracellular domains comprising the nt 273-4184 of the above sequence is represented in SEQ ID NO: 1.

The term "ER targeting signal peptide" or "leader sequence" relates to an amino-terminal (N-terminal) signal sequence mediating the targeting of a nascent secretory and membrane protein to the endoplasmic reticulum (ER). An example for such an ER targeting signal peptide is a CD8 leader sequence. A nucleotide sequence encoding such a CD8 leader sequence is presented in SEQ ID NO: 13.

The term "membrane anchoring domain" as used herein means a carboxy-terminal (C-terminal) amino acide sequence with which a protein is anchored in a cell membrane. The term here comprises the term "GPI-addition signal peptide" (GPIsp), relating to a C-terminal amino acid sequence signaling the addition of a GPI anchor to the partially folded nascent protein. Usually, the GPIsp is removed and directly replaced by a GPI precursor, which is further processed, i.a. by carbohydrate side-chain modifications, to the final GPI anchor. The term "GPI anchor" relates to a glycosylphosphatidylinositol structure having an ethanolamine phosphat residue, with which the structure is covalently bound to the C-terminal of a protein, and being anchored in a cell membrane via a lipid component, e.g. an inositolphospholipid [see e.g. [9]). Most GPI anchors have the following core structure: Manα1-4GlcNα1-6myo-inositol-1-PO₄-lipid, e.g ethanolamine-PO₄-6Manα1-2Manα1-6Manα1-4GlcNα1-6myo-inositol-1-PO₄-lipid. This core structure may be modified by various substitutions. An example of a coding sequence for a "GPI-addition signal peptide" is given in SEQ ID NO: 14.

The term "murine animal" refers to animals of the subfamily Murinae in the family Muridae. In particular, the term refers to animals of the genus Mus and Rattus. Examples for murine animals are animals of the species Mus musculus (house mouse) and Rattus norvegicus (brown rat, Norway rat). In case the current invention refers to crossing steps between murine animals, it is understood that murine animals are meant being able to produce fertile offspring. In particular, crossing a murine animal with another murine animal therefore means crossing murine animals of the same species. The terms "mouse" and "rat" or "laboratory mouse" and "laboratory rat" used herein are meant to refer to the house mouse (Mus musculus) and brown rat (Rattus norvegicus), respectively.

In a preferred embodiment of the invention the membrane anchoring domain is or comprises a GPI-addition signal peptide domain, and/or the ER targeting signal peptide is or comprises a human CD8 leader sequence. Preferably, the GPI-addition signal peptide domain is encoded by a nucleic acid having the sequence of SEQ ID NO: 14.

In a further preferred embodiment of the invention the nucleic acid further encodes a reporter protein. A reporter gene is a gene not natively present or expressed in a cell under study and which is introduced and expressed together with the gene of interest, thus functioning as a marker for the successful uptake and/or expression of the gene of interest. For this purpose, a reporter gene is usually arranged in immediate vicinity of the the gene of interest. It may be placed under the control of the same promoter as the gene of interest. An example of a preferred reporter gene is eGFP (enhanced Green Fluorescent Protein), causing cells expressing it to glow green under blue light. In the nucleic acid the eGFP coding sequence is preferably located downstream of the fusion protein coding sequence. The reporter gene sequence may be preceded by an internal ribosome entry site (IRES), i.e. a nucleotide sequence being able to bind the eukaryotic ribosome, thus allowing translation initiation to occur in the middle of a messenger RNA (mRNA).

In a further preferred embodiment, the genetically engineered murine animal of the invention is a murine animal being transfected with a vector comprising or having a sequence according to SEQ ID NO: 2. The vector represented by SEQ ID NO: 2 is a modified Rosa26-CAG-Stop-eGFP vector (No.: 15912, Addgene, Cambridge, Ma, USA; [5]), containing a strong chicken actin promoter (AProm), a floxed neomycin-stop cassette (Stop), a nucleic acid coding for a hPLA2R/GPI fusion protein and an IRES triggered fluorescence reporter protein (GFP), which specifically integrates into the mouse Rosa26 (R26) locus through its R26 sites allowing targeted integration by homologous recombination.

In a second aspect the invention relates to a genetically engineered murine animal, preferably a mouse or rat, obtainable by transfecting an embryonic stem cell of a murine animal with a vector comprising a nucleic acid encoding a fusion protein comprising the extracellular domains of hPLA2R, an ER targeting signal peptide and a membrane anchoring domain, integrating the transfected embryonic stem cell into a blastocyst of a non-transfected murine animal, crossing the chimeric animal resulting from the blastocyst with a non-transfected murine animal and selecting a transfected murine animal from the offspring. The ER targeting signal peptide is preferably a CD8 leader sequence, e.g. being encoded by nucleic acid having a sequence of SEQ ID NO: 13, the membrane anchoring domain is preferably a GPI-addition signal petide, preferably being encoded by nucleic acid having a sequence of SEQ ID NO: 14. Preferably, the nucleic acid further encodes a reporter protein, for example eGFP. The reporter protein coding sequence is preferably located downstream of the fusion protein coding sequence.

It is known to the skilled person how to transfect embryonic stem cells of murine animals and how to produce murine animals therefrom, harboring a transgene in all of its germ and somatic cells. A vector may, for example, be introduced into a pluripotent embryonic stem cell by electroporation. The introduced vector than is inserted into the genome by homologous recombination, thus, e.g. "knocking in" a functional gene into a desired locus. Transfected cells are placed in a selective medium and surviving clones are isolated and grown in culture. Cells tested positive for homologous recombination, e.g. via a reporter gene product, are injected into blastocysts from a donor murine animal, e.g. a laboratory mouse, e.g. a C57BL/6 mouse, and implanted into pseudopregnant foster mothers. Chimeric offspring are backcrossed, e.g. with C57BL/6 females, to generate heterozygous animals. Heterozygous animals are then intercrossed to generate homozygous gene-targeted animals.

Suitable vectors are known in the art, and include, for example, Rosa26 vectors, i.e. vectors targeting the genomic mouse locus Rosa26 in order to knockin e.g. cDNA constructs for ubiquitous or conditional gene expression in transgenic mice. A exemplary Rosa26 vector is a CAG-STOP-eGFP-ROSA26 targeting vector (e.g. plasmid 15912, Addgene, Inc., Cambridge MA 02139, USA; [5]), CAG standing for the CAG promoter (chicken beta-actin promoter with CMV enhancer).

Especially preferred, the genetically embryonic stem cell is transfected with a vector comprising or having a sequence according to SEQ ID NO: 2.

In a further aspect the current invention relates to a genetically engineered murine animal, preferably a mouse or rat, further preferred a mouse, expressing exclusively in its podocytes a fusion protein comprising the extracellular domains of the human phospholipase A2 receptor (hPLA2R), an ER targeting signal peptide and a membrane anchoring domain. In this embodiment the human PLA2 receptor is essentially exclusively expressed in the podocytes of kidney glomerula of the murine animal of the invention, which thus represent an animal model coming very close to the situation in humans. Preferably, the nucleic acid further comprises a coding sequence for a reporter protein, e.g. eGFP, preferably downstream of the fusion protein coding sequence.

Exclusive expression of the fusion protein in podocytes can, for example, be achieved by a recombinase, being expressed in a podocyte-specific manner, e.g. under the control of a podocyte-specific promoter, the recombinase removing a stop cassette present in a nucleic acid and preventing the expression of the fusion protein comprising the extracellular domains of the human phospholipase A2 receptor (hPLA2R), the ER targeting signal peptide and the membrane anchoring domain.

The genetically engineered murine animal of this aspect of the invention may be obtained by crossing a murine animal according to the first aspect of the invention, or a murine animal according to the second aspect of the invention, with a genetically engineered murine animal expressing a recombinase exclusively in its podocytes. An example for a suitable recombinase is the Cre recombinase. The Cre recombinase is a 38kDa tyrosine recombinase enzyme derived from the bacteriophage P1 catalyzing the site specific recombination between two DNA recognition sites (loxp sites). Preferably, the genetically engineered murine animal is a mouse. Transgenic mice that express Cre recombinase exclusively in podocytes are known (see [6]).

In still another aspect the invention relates to a process for manufacturing a genetically engineered murine animal comprising the steps of transfecting an embryonic stem cell of a murine animal, preferably a mouse or rat, further preferred a mouse, with a vector comprising a nucleic acid encoding a fusion protein comprising the extracellular domains of the human phospholipase A2 receptor (hPLA2R), an ER targeting signal peptide and a membrane anchoring domain, integrating the embryonic stem cell into a blastocyst of a non-transfected murine animal, crossing the chimeric animal resulting from the blastocyst with a non-transfected murine animal and selecting a transfected murine animal from the offspring.

In a preferred embodiment the process further comprises the step of crossing the transfected murine animal produced with a genetically engineered murine animal expressing a recombinase, preferably a Cre recombinase, exclusively in its podocytes.

### {Examples}

### {1. General description of the generation of the hPLA2R-Knockin mice of the invention.}

In a first step, a nucleic acid encoding a fusion protein was generated, consisting of the complete extracellular part of the human PLA2R (NCBI accession number: NM_007366; bp 273-4184; [2]; SEQ ID NO: 1), a GPI-anchor domain for efficient integration into cell membranes [3] and a CD8-leader sequence used in other artificial systems [4]. To generate this fusion protein the pCMVSport6-MfeDANOD-KV-FS vector (DANOD, [4]) containing the CD8-leader as well as the GPI-anchor domain was used as the primary vector. The complete extracellular human PLA2R domains were specifically amplified by PCR from a commercially available full-length cDNA construct (No. SC308948, OriGene, Rockville, MD, USA). Subsequently, this PCR fragment was cloned in frame between the CD8-leader and the GPI anchor (1.1).

In a second step the nucleic acid coding for the fusion protein produced in step one was subcloned into a knockin target vector, called Rosa26-CAG-Stop-eGFP (No.: 15912, Addgene, Cambridge, Ma, USA; [5]) to obtain a hPLA2R/GPI carrying construct (figure 1, 1.2.). This modified target vector specifically integrates into the mouse Rosa26 (R26) locus through its R26 sites that allow targeted integration by homologous recombination. Furthermore, this vector contained a strong chicken actin promoter (AProm), a floxed neomycin-stop cassette (Stop), the nucleic acid coding for the hPLA2R/GPI fusion protein and an IRES triggered fluorescence reporter protein (GFP). R1-ES-cells (1.7.) were transfected with the hPLA2/GPI construct (1.8.) to obtain pluripotent ES cells having stably integrated foreign DNA into their R26 locus. Next, these ES cells were injected in blastocysts while the modified blastocysts were implanted in false pregnant females to create chimeric littermates (1.14.). Further breeding of the chimera with C57BL/6 females resulted in littermates with a stable hPLA2R/GPI positive genotype. In the following, these animals are called "hPLA2R/GPI" mice.

For podocyte-specific protein expression the hPLA2R/GPI mice were finally bred with Podo-Cre mice [6]. These mice express the Cre recombinase under the control of the podocine promoter. Due to this highly specific promoter the Cre recombinase expression is strictly confined to podocytes. Consequently, the floxed stop-cassette is removed exclusively in podocytes and the strong actin promoter activates transcription of hPLA2R/GPI as well as eGFP, leading to translation of hPLA2R/GPI and eGFP (figure 2). These mice specifically express hPLA2R in their podocytes (figure 3), and are therefore called "Podo-Cre hPLA2R/GPI" mice. These mice are especially useful for establishing human PLA2R-dependend MN models.

### {2. Detailed description of the generation of the hPLA2R-Knockin mice of the invention.}

### {2.1 Generation of the GPI-PLA2R fusion construct}

A sequence-verified construct of the full-length PLA2R cDNA (NM_007366, TrueClone No.: SC308948, OriGene,) was used as the source to amplify the extracellular part [2] of the human PLA2R by PCR. The PCR-fragment was cloned into the DANOD-vector [4].

For cloning, cut 5 µg of the DANOD vector with Mfe-I (No.: R0144; NEB, Ipswich, MA, USA) and Bgl-II (No.: R0589, NEB) in NEB-buffer 2 at 37° C over night (10 U of each enzyme in 50 µl). Separate the vector-backbone from the former insert in a 1 % agarose/TAE gel at 90 V until the fragments are clearly distinguishable. Cut out the vector by a scalpel and purify the DNA through the QIAquick Cell Extraction Kit (No.: 28704, Qiagen, Hilden, Germany) according to the manufacturer's recommendations.

For PLA2R-specific PCR amplification, use 5 ng of the original construct (No.: SC308948 Origene) add specific forward (FW) and reverse (Rev) DANOD primers containing Bgl-II or Mfe-I restriction-sites at their 5'ends respectively (3.1.1.), deoxynucleotide-mix (8 mM, No.: R0241, Fermentas/Fisher Scientific, Schwerte, Germany), Phusion-DNA-polymerase with proof-reading activity (No.: F-530, Fermentas) and specific 5x polymerase-buffer in a total volume of 50 µl. Use the following amplification-protocol in a thermal cycler (LifeTouch, Biozym, Hessisch Oldendorf, Germany): Once 3 minutes, 98° C; 35x 15 s, 98° C; 15 s, 66° C; 6 minutes, 72° C and a final extension step for 6 minutes at 72° C. Add 10 U of Mfe-I and Bgl-II respectively as well as 10x NEB-buffer 2. Directly cut the PCR fragment (60 µl) for 2 h at 37° C in a water bath (U3, Julabo, Seelbach, Germany). Purify the fragment using the PCR Purification Kit (No.: 28106, Qiagen). Check the integrity of both DNA-fragments in a 1 % agarose/TAE gel and estimate the approximately DNA concentration by comparing the intensity of each fragment with the known concentrations of the DNA-ladder bands (1 kb Plus DNA Ladder No.: 10488095 Life Technologies, Darmstadt, Germany).

Ligate 100 ng vector-DNA with different molar ratios of the PCR-fragment (3:1, 1:1, 1:3) and 1 U T4-DNA Ligase (No.: M0202, NEB) in specific 10x ligation-buffer containing 10 mM ATP in 10 µl at 12° C over night by using a thermal cycler (LifeTouch, Biozym). Add 90 µl 1xTE and perform transformation with 5 µl ligation-reaction/30 µl chemical competent XL10 E. coli (No.: 200315, Agilent Technologies, Santa Clara, Ca, USA) for 30 minutes on ice. Then heat shock E. coli 45 s at 42° C, put on ice 2-3 minutes, add 220 µl LB-Medium (No.: 3002-031, MP-Biomedical, Solon, OH, USA) and incubate E. coli for 1 h at 37° C in a shaking-incubator (GFL). Strike out the bacteria suspension on LB-Agar plates supplemented with 100 µg/ml ampicillin (No.: 8495222, Böhringer Mannheim, Mannheim, Germany) and incubate these plates over night at 37° C (B 6030, Kendro/Heraeus, Langenselbold, Germany) for selection of vector-DNA containing, ampicillin resistant colonies.

Pick some colonies with a toothpick, transfer to 50 µl 1 x TE and mix thoroughly. Identify positive, entire vector-construct carrying E. coli by screening PCR. Mix together (20 µl end volume) 5 µl E. coli suspension, 1 µl of each FW- and Rev-DANOD Screening primers (10 mM, 4.1.2.), 0.4 µl deoxynucleotid mix (8 mM, Fermentas), 0.2 µl Dream-Taq polymerase (No.: EP0712, Fermentas) and 2 µl 10x Dream-Taq buffer. Use the amplification protocol in a thermo cycler (LifeTouch, Biozym) as follows: Once 5 minutes, 95° C; 40x 30 s, 95° C; 30 s 64° C; 6 minutes 72° C and once 6 minutes 72° C for final extension. Check the fragments in a 1 % agarose gel at 90 V for 45 minutes. Transfer positive clones with a 10 µl pipet-tip in LB-Medium (50 ml) and incubate for about 1 day at 37° C in a shaking incubator (GFL). Isolate vector-DNA using the PureLink Plasmid Midi Kit (No.: K210005, Life Technologies) according to the manufacturer's recommendations. Further check integrity by restriction enzyme digestion (Mfe-I/Bgl-II) and complete sequencing of the cloned construct including CD8-leader and GPI-anchor.

### {2.2. Generation of the R26 PLA2R-specific Target Vector}

A clone without any mutations and correct in frame cloning of the CD8 leader and GPI-anchor was used as starting material (2.1.). There is one Asc-I restriction site to clone the foreign-DNA of interest between the floxed stop-cassette and the GFP-reporter. Therefore, the complete fusion cDNA construct was re-amplified from the DANOD vector by PCR.

Cut 5 µg of the R26-Stop-eGFP Vector [5] with 25 U Asc-I (No.: R0558, NEB) in 10x NEB-buffer 4 in 50 µl at 37° C over night. Add 10 U calf intestine alkaline phosphatase (No.: M0290, NEB) for 1 h at 37° C to dephosphorylate the vector ends. Add 50 µl 10 mM Tris/Cl pH 8.0 and perform phenol (Tris/Cl pH 8.0 saturated, No.: A980-1, C. Roth, Karlsruhe, Germany) extraction twice. Add 10 µl 3 M Na-Acetate pH 5.2 (No.: 1.06267, Merck, Darmstadt, Germany) and 300 µl 100 % ethanol (No.: 9065.1, C. Roth) to precipitate DNA 1-2 h at -80° C. Centrifuge 20 minutes at 20,000xg (4° C, 5417R, Eppendorf, Hamburg, Germany) and wash the pellet three times in 70 % ethanol, dry 3-5 minutes in a Speed Vac (Concentrator 5301, Eppendorf) and dissolve in 12 µl 10 mM Tris/Cl pH 8.0. Estimate DNA concentration on a Nano Drop (ND-2000, Peqlab, Erlangen, Germany).

For PCR amplification, use 5 ng of the DANOD-construct, add specific FW and Rev R26-primers containing Asc-I restriction-sites at their 5'ends (3.1.3.) All other components are identical as used in chapter 2.1. "PLA2R-specific amplification". Add 10 U Asc-I in 10x NEB-buffer 4 for 2 h at 37° C.

Ligation, Transformation, PCR-screening as well as the final verifications are performed as mentioned in chapter 2.1., with the exception that R26-Screening FW and Rev primers were used (3.1.4., SEQ ID NO: 9, 10).

Finally, linearize 70 µg verified PLA2R-specific target vector with 100 U AsiSI (No.: R0630, NEB) at 37° C over night in 300 µl 10 mM Tris/Cl pH 8.0. Purify DNA through one phenol (C. Roth) and one chloroform (No.: 4423.1, C. Roth) extraction and precipitate DNA by adding 30 µl 3 M Na-Acetate pH 5.2 (Merck) and 1 ml 100 % ethanol (C. Roth). Further steps see 2.1. Invert the tube several times during the extractions because the 20 kb fragment is very sensitive. The linearized DNA is now ready to be transfected in ES-cells (2.8.). The sequence of the target vector is presented in SEQ ID NO: 2.

### {2.3. Generation of Mouse Embryo Fibroblasts (MEF)}

Use 13.5-14.5 dpc (day post coitum) old embryos of FVB mice. For this purpose three pregnant females (8-10 embryos/female) are required.

Dissect the uterus, wash out the embryos with 1 x PBS and remove the embryo's envelope. Remove all organs and intensively wash the torso with 1 x PBS. Transfer the torso in 2-3 ml of suspension-medium to a new Petri dish, mince it by a scalpel and press the shredded tissue through 20 G and 26 G cannulas to complete homogenization. Put the homogenate into a 15 ml tube, add a small amount of DNase powder (No.: D-4527, Sigma) and 5 ml of 0.05 % Trypsin/EDTA. Incubate for 10 minutes at 37° C, mix occasionally. Let the suspension settle down for one minute and collect the supernatant in 20 ml MEF-Medium. Digest the remaining suspension again for one minute in 5 ml trypsin mixture, put the supernatant to the first and repeat this step 2-3 times. Count the cells (exclude the small erythrocytes) and cultivate 5 x 10⁶ cells/15 cm Petri dish (No.: 639160, Greiner, Frickenhausen, Germany) in 25 MEF-Medium supplemented with 15 % FCS. Change the medium every day. Three to four days later the MEF should be confluent. Split 1:3-1:5 or freeze 2.5 x 10⁶ MEF in 2 x Freeze (see 3.10).

### {2.4. Gelatin treatment}

Incubate dishes with 0.1 % gelatin for 30 minutes at room temperature. Be sure that the bottom of the different dishes is totally covered by the liquid (the volume depends on the diameter of the dish), discard the solution and let them air dry. The dishes can be stored at 4°C.

### {2.5. MEF cell culture}

Quickly thaw 2.5 x 10⁶ MEF at 37° C in a water bath, dilute in 25 ml MEF-Medium and cultivate them for 3 days until confluence in a 15 cm Petri dish (Greiner). To split the MEF 1:3 to 1:4 discard medium and wash with 25 ml 1 x PBS, add 3 ml of 0.05 % Trypsin/EDTA for 3 - 5 minutes at 37° C. Pipette up and down until cells are scattered. Stop digestion by adding 12 -17 ml MEF-Medium (depends on dilution ratio), thoroughly mix the suspension and put 5 ml of this suspension to a new plate equipped with 15 ml MEF-Medium. Let them grow another 3 days.

### {2.6. Inactivation of MEF cells}

Replace the medium (15 ml) of a confluent 15 cm MEF-plate, add 150 µl Mitomycin C (10 µg/ml) and incubate 2-3 h at 37° C. Discard the medium and wash twice with 20 ml 1 x PBS. Add 3 ml of 0.05% Trypsin/EDTA for 3-5 minutes, pipette up and down, stop digestion with 7 ml MEF-Medium. Collect the suspension and count cells. Centrifuge and suspend the MEF 1-5 x 10⁶/ml freeze medium on ice. Freeze the inactivated MEF in 1 x Freeze or dilute to 5 x 10⁴ MEF/ml in MEF-Medium and cultivate them in gelatinated Petri-dishes for ES cell cultures.

### {2.7. ES cell culture}

R1-ES cells were obtained from Prof. Nagy, Samuel Lunenfeld Research Institute, Mount Sinai Hospital Toronto. They were obtained at passage 11 and expanded to passage 14 and 15, respectively. For details of ES-strain see: http://www.informatics.jax.org/mgihome/nomen/strain_129.shtml. Alternatively, R1/E cells ATCC SCRC-1036 (LGC Standards GmbH, Mercatorstr. 51, 46485 Wesel, Germany; http://www.1gcstandards-atcc.org) could be used.

Thaw ES-cells quickly in a water bath at 37° C, dissolve them in 5 ml ES medium and centrifuge cell-suspension 5minutes at 300xg at room temperature. Suspend the ES-cell pellet in ES medium (the volume depends on the number of frozen cells) and plate on gelatinated cell culture plates supplemented with a confluent layer of inactivated MEF cells (feeder cells) Change the ES medium every day and split confluent ES-cells in a ratio of 1:4 to 1:6 every second day. To perform splitting, wash cells with 1 x PBS/EDTA and subsequently trypsinize with 1/5 volume of 0.25 % Trypsin/EDTA for 3-5 minutes at 37° C. Pipette cells up and down to get a homogeneous single cell suspension. Stop trypsin activity by adding 2 x volumes of ES medium and centrifuge for 3 minutes at 300xg at room temperature. Suspend the pellet in the appropriate volume of ES medium depending on the cell culture plate and dilution factor of choice. Plates must be gelatinated and plated with an appropriate number of inactivated MEF the day before use.

### {2.8 Electroporation and Selection of ES-Cells}

Use 1 x 10⁷ ES-cells/electroporation and change ES medium 2 h before trypsinization. After trypsination (2.7) suspend the pellet in 10 ml 1 x PBS and centrifuge for 3 minutes at 300xg (room temperature). Suspend the pellet in 0.8 ml 1 x PBS, add linearized DNA (25-80 µg, 2.2.) and transfer into a 0.4 cm gap-electroporation-cuvette (No.: 65-0032, Life Technologies/Gibco). Use the Electroporator (Gene Pulser II, Biorad, Hercules, CA, USA) with the settings as follows: Double-pulse 250 V, 500 µF. Store electroporated cells 10 minutes at room temperature and transfer them into 10 ml ES medium before spreading on 5-6 10 cm dishes (No.: 664160, Greiner, gelatinated + inactivated MEF). For positive selection change ES medium supplemented with 150-300 µg/ml Geneticin (G418, Life Technologies/Gibco) every day for one week. During this week G418 resistant ES cell clones (colonies) should grow that stably incorporate the foreign DNA in their genome. Pick the colonies with 10-20 µl pipet-tips by using a binocular (SZH10 equipped with DF PLANAPO IX, Olympus, Hamburg, Germany) transfer each colony into one well of a round bottom 96-well plate (650180, Greiner) supplemented with 40 µl 0.25 % trypsin solution and incubate 3-5 minutes at 37° C. During this incubation replace the MEF-Medium with 160 µl of ES medium in a gelatinated, feeder cell layered flat bottom 96-well plate (550180, Greiner). Afterwards transfer the total amount of trypsinized and singulated cells to the new plate and mix suspension by pipetting up and down carefully. 2-3 days later the cells should be confluent. For splitting, wash the well with 200 µl 1 x PBS/EDTA and trypsinize with 40 µl 0.25 % trypsin for 3-5 minutes at 37 °C, pipette up and down until cells are single. Stop enzyme activity by adding 160 µl of ES medium. Distribute cell-suspension on two gelatinated, feeder cell layered flat bottom 96-well plates as well as one gelatinated 96-well plate without any feeder cells. Again, 2-3 days later the cells should be confluent. Freeze both plates that include feeder cells and prepare the other plate without feeders for DNA-isolation.

### {2.9. Freeze of ES cells}

Change ES medium 2 h before freezing. Wash each well with 200 µl PBS/EDTA, trypsinize the cells with 40 µl 0.25 % trypsin for 3-5 minutes at 37 °C, pipet up and down, stop enzyme-activity by adding 60 µl ES medium. Put the plate on ice, add 100 µl 2 x Freeze and gently mix. Overlay suspension with 50 µl mineral-oil, wrap the plate with adhesive tape and freeze in a Styropor-box at -80 °C. Two days later remove the box and store the plates at -80 °C.

### {2.10. DNA-Preparation}

Discard ES medium of confluently grown ES cell layers, wash with 200 µl 1 x PBS and discard. Add 100 µl DNA lysis buffer and incubate over night at 55 °C in a moist chamber. Add 10 µl 8 M LiCl and 100 µl 2-propanol. To precipitate DNA, gently shake the plate over night in a moist chamber (Important: Tightly seal the plate with parafilm). Centrifuge the plate for 30 minutes at 200xg at 4 °C. Discard supernatant and wash twice with 70 % ethanol (centrifuge between the washes, 20 minutes, 2000xg, 4 °C).

### {2.11. Southern Blot, Random-Prime-Labeling and Hybridization}

Run a 1 % agarose/TAE gel with EcoRI digested DNA-samples (see 2.10.) using DNA loading buffer. Use fresh 1 x TAE Bbffer and a clean chamber. To facilitate transfer of DNA-fragments longer than 3 kb incubate the gel for 5 minutes in 0.25 N HCl at room temperature, discard HCl and wash extensively with deionized water. Add 0.4 N NaOH and incubate for 15 minutes. Prepare filter papers (5 pieces) and the XL-1 membrane for blotting and moisten them with 0.4 M NaOH. Put the gel onto a glass-plate, then place the Hybond N+ membrane (No.: RPN119B, GE Healthcare/Amersham, Freiburg, Germany) and the filter papers without any air-bubbles on the gel. Put a stack of dry paper-towels then a second glass plate onto the filter papers and wrap the whole with saran wrap and place a weight at the top. Incubate over night at room temperature. Float membrane with 2 x SSC not more than 10 minutes and bake it for 2 h at 80 °C.

Label the DNA probe (Rosa probe-A 693 Bp, EcoR-I/Pac-I fragment) [5, supporting online materials] with alpha 32P dATP using the Megaprime DNA Labeling System (No.: RPN1604, GE Healthcare/Amersham) according to the manufacturer's recommandations.

For hybridization put membrane tightly in a hybridization bottle, add pre-warmed hybridiziation buffer and roll it 1-2 h at 68 °C. Discard buffer, cook the random prime labeled probe (2.5 x 10⁵ - 5 x 10⁵ cpm/ml) for 5 minutes, add fresh hybridization buffer (about 5 ml, depends on the bottle) including the labeled DNA probe and let it roll over night.

Discard hybridization buffer and wash the membrane in 2 x SSC 0.5 %SDS. Pull out the membrane and wash it in 2 x SSC 0.5 % SDS for 15 minutes at 68 °C. Discard wash buffer and repeat once. Further wash membrane in 0.2-0.1 x SSC several times for 10-15 minutes until no radioactivity can be detected in the wash buffer. Put the membrane between saran wrap and expose.

### {2.12. Generation of blastocysts}

Sacrifice donor mice (3.5 dpc) and place on back. Spray abdomen with skin disinfectant solution (Braunoderm^{®}, B. Braun Melsungen AG, Melsungen, Germany). Pick up the skin with a blunt forceps, cut through the skin, pull at both ends of the cut to expose the internal fascia and then cut through the internal fascia. Expose uterine horns, ovaries, oviduct and cervix and pick up the fat pad on the ovary on one side of the mouse. Remove the fatty mesoterium along the uterus on both sides and then cut it out. Place the uterus in a dish containing 1 x PBS. Place the uterus on a lid of a 10 cm dish (Greiner) under the binocular (Olympus) and remove any tissue and fat which may be still attached to the uterus. Then cut of the oviducts, insert an eye scissor in the opening of the uterus to widen it. Place the uterus for an additional wash in a new dish with 1 x PBS. Fill a 1 ml syringe with KSOM/HEPES attach a 30 G needle, place the uterus cervix facing towards you on the edge of a depression dish under the binocular (Olympus), insert the needle through the cervix. Rinse each horn with 0.5 ml of medium. Collect the blastocysts with a mouth controlled glass capillary and place them in a drop or KSOM covered with mineral oil at 37 °C, 5 % CO₂.

### {2.13. Injection of ES cells in blastocysts}

Start with a confluent well (6-well plate, No.: 657160, Greiner) of the desired ES cell clone, change ES medium 2 h prior trypsination. Wash cells with 3 ml 1 x PBS, add 0.5 ml 0.25 % trypsin and incubate for 2-3 minutes at 37 °C, pipette up and down gently. Stop enzyme reaction in 5 ml ES medium. Don't mix the solution. At this stage 2-10 cell aggregates are recommended. Centrifuge 3 minutes at 300xg, carefully suspend the pellet in 0.8 ml HEPES buffered ES medium and store on ice at least 30 minutes.

Prepare injection set up (Olympus IX70; Eppendorf: PatchMan NP2, TransferMan NK2, Cell tram air, Cell tram vario), i.e. turn on cooling water, fill holding pipette with 1 x PBS, make sure the oil in the injection capillary is smoothly moving. To start, fill injection slide with 0.5 ml cold KSOM/HEPES flick tube with the cells and pipette 3-6 blastocysts on the upper part of the injection slide. Add 50 µl of the ES-cell suspension to the lower part of the injection slide, put the slide on the microscope stage and bring both capillaries in the medium (not to the bottom). Start to collect ES-cells with clearly visible, small, round nuclei without any vacuoles and inject 15 ES cells per blastocyst. Use the holding pipette to fetch and fix a blastocyst. Turn the fixed blastocyst until you can inject the ES-cells between the border of trophoblast without hurting the inner cell mass. Place the ES cells on the top of the inner cell mass. Soak of additional volume carefully and place the injected blastocyst separate from the others. As soon as the blastocysts on the slide are injected transfer them to the incubator (Innova CO-14, Eppendorf), in a pre-equilibrated (37 °C) drop of KSOM.

### {2.14. Implantation of blastocysts}

Female mice mated with vasectomized males at 2.5 dpc are used. Weight of the mouse is determined. Injection of Ketamin/Rompun for anesthesia: 120/16 mg/kg KG. Check pedal reflex, inject Rimadyl (5 mg/kg s.c.) for analgesia. Shave back carefully, wipe with tissue soaked with Braunoderm. Put mice on a clean Kimwipe and place it under the binocular (Olympus) on a heating mate. Make a small incision on the back parallel to the last rib. Wipe incision with a fresh tissue soaked with Braunoderm and slide the skin laterally until the ovary can be seen under the fascia. Make a small incision over the ovary and grap the ovaries fat pad and use a blunt forceps to pull fat pad, ovary and the upper part of the uterine horn. Clamp the fat pad with a serrafine and place it and the ovary and uterus on a small sterile filter paper. The corpus luteum must be clearly visible otherwise the mouse is not pseudo pregnant. Use a mouth pipette with glass capillary attached to transfer the blastocysts to a fresh drop of medium and then soak up to 8 of them in the capillary like pearls on a string. Gently grap the uterine horn with a fine forceps, take a 27 or 30 G needle and make a hole in the uterus. Insert the capillary containing the blastocysts and slowly expel them into the uterus. Withdraw the capillary, release the serrafine and put the fat pad, ovary and uterus back. Close the inner skin with sutures and repeat on the other side. Clip the outer skin with wound clips, disinfect using Braunoderm, wrap mouse in a sterile tissue and place it in a fresh type 2 long cage on a heating mat until it is awake and moving. Health control is done daily up to five days before delivery, cages and water is changed once a week. 5 days before delivery mice are placed in a fresh cage, with lots of nesting material and their old nest. Five days after delivery pups are counted and sexed and after five additional days chimerism can be determined. They are mated to wildtype females as soon as they are mature. Brown offspring is then genotyped.

### {2.15. PLA2R-GPI genotyping}

Isolate genomic DNA from small pieces of tail-tissue in 150 µl of Direct-PCR-Tail (No.: 31-102T, Peqlab) supplemented with 0.25 mg/ml Proteinase K (4.12.) at 55° C over night. Stop reaction at 85° C for 45 minutes.

Add 1 µl of gDNA-solution, 1 µl of PLA2R/GPI-genot. FW and Rev. primers respectively (10 mM each; 3.1.5.), 0.4 µl dNTP mix (8 mM, Fermentas) and 0.2 µl Dream-Taq (Fermentas) in the appropriate buffer in a total volume of 20 µl. Use the amplification protocol in a thermo cycler (LifeTouch, Biozym Scientific GmbH, 31840 Hessisch Oldendorf, Germany) as follows: Once 5 minutes, 95° C; 40 x 30 s, 95° C; 30 s 64 °C; 1 minutes 72 °C and once 3 minutes 72 °C for final extension. Check the fragments in a 1.5 % agarose gel at 90 V for 45 minutes.

### {3. Material}

### {3.1. PCR-Primers}

{3.1.1. DANOD-Primers:}
FW: 5' TTTTTTAGATCTGAGGGTGTGGCGGCGGCGCTT 3' (SEQ ID NO: 3)
Rev: 5' TTTTTTCAATTGTTCTTACTGTTACCATCAAATTGAGC 3' (SEQ ID NO: 4)
{3.1.2. DANOD-Screening-Primers:}
FW: 5' CTTGCTCCTGCCGCTAGCCTT 3' (SEQ ID NO: 5)
Rev: 5' CAAGGAACAGGGATACACAGCTT 3' (SEQ ID NO: 6)
{3.1.3. R26-Primers:}
FW: 5' AAAAAGGCGCGCCTTAGCGATGGCCTTACCAGTGACCGCCT 3' (SEQ ID NO: 7)
Rev: 5' AAAAAAGGCGCGCCCTACGGCTCAGCAAGACAAAGCA 3' (SEQ ID NO: 8)
{3.1.4. R26-Screening-Primers:}
FW: 5' AATAGAACTCTTGCTTGCTTTGCTA 3' (SEQ ID NO: 9)
Rev: 5' TCTTCAAGAAGCTTCCAGAGGAA 3' (SEQ ID NO: 10)
{3.1.5. PLA2R/GPI genot.-Primers:}
FW: 5' TTAACAATCAAGGATGAGGCT 3' (SEQ ID NO: 11)
Rev: 5' TCTTCAAGAAGCTTCCAGAGGAA 3' (SEQ ID NO: 12)
{3.2 50x TAE buffer:}
Tris base 242 g, dissolve in 750 ml deionized water
+ 57.1 ml glacial acetic acid
+ 100 ml 0.5 M EDTA pH 8.0
adjust volume to 1 L
working solution is 1x
{3.3 1x TE:}
10 mM Tris/Cl pH 8.0
1 mM EDTA pH 8.0
{3.4 10x PBS:}
10.9 g Na₂HPO₄ anhydrous
+ 3.2 g NaH₂PO₄
+ 90 g NaCl
dissolve just under 1 L deionized water, then adjust pH to 7.4
working solution is 1 x
{For PBS/EDTA:}
500 ml 1 x PBS
+ 0.1g EDTA
{3.5. Trypsin:}
Trypsin/EDTA MEF cells (0.05 %, No.: 25300054 Life Technologies/Gibco)
Trypsin/EDTA ES cells (0.25 %, No.: 25200056 Life Technologies/Gibco)
{3.6. MEF Medium:}

| | | |
|---|---|---|
| DMEM No.: 6196, 5026 Life Technologies/Gibco | | 500 ml: |
| FCS (Gold) No.: A11-649, PAA | 9% | 50 ml |
| MEM (100x) No.: 111400 Life Technologies/Gibco | 1x | 5.5 ml |
| Pen/Strep (100x) No.: 15140122 Life Technologies/Gibco | 1x | 5.5 ml |

{3.7. ES medium:}

| | | |
|---|---|---|
| DMEM, No.: 42430025 Life Technologies/Cribco | | 500 ml: |
| FCS (Gold), A11-649 PAA | 15% | 92.0 ml |
| Glutamin (100x), No.: 25030024 Life Technologies/Gibco | 1x | 6.2 ml |
| MEM (100x), No.: 11140035 Life Technologies/Gibco | 1x | 6.2 ml |
| Pen/Strep (100x), No.: 15140122 " | 1x | 6.2 ml |
| Nucleosidmix (100x), see 4.8. | 1x | 6.2 ml |
| Sodium pyruvate, No.: 1136003 " | 1x | 6.2 ml |
| 2-Mercaptoethanol (50 mM), No.: 31350010 " | 2x | 1.24 ml |
| LIF (107 U/ml), No.: ESG 1107 Chemicon Int. | 1000 U/ml | 62 µl |
| For selection add: | | |
| G418 (50 mg/ml) No.: 10131019 Life Technologies/Gibco | | 4-6 µl |

{3.8. Nucleoside Mix 100x:}

| | | |
|---|---|---|
| Adenosine | No.: A-4036 Sigma | 80 mg |
| Guanosine | No.: G-6264 Sigma | 85 mg |
| Cytidine | No.: C-4654 Sigma | 73 mg |
| Uridine | No.: U-3003 Sigma | 73 mg |
| Thymidine | No.: T-1895 Sigma | 24 mg |

{3.9. Electroporation buffer:}

| | |
|---|---|
| HEPES | 20 mM |
| NaCl | 137 mM |
| KCl | 5 mM |
| Na₂HPO₄ | 0.7 mM |
| Dextrose | 6 mM |
| Adjust pH to 7.05 using 0.5 N NaOH | |

{3.10. Freeze media:}

| | |
|---|---|
| 1 x Freeze: | |
| FCS | 40 % |
| Appropriate Medium | 50 % |
| DMSO No.: D-2650, Sigma | 10 % |

{2 x Freeze:}

| | |
|---|---|
| FCS | 80 % |
| DMSO | 20 % |

{3.11. DNA lysis buffer:}

| | |
|---|---|
| Tris/Cl pH 7.5 | 10 mM |
| EDTA | 10 mM |
| NaCl | 10 mM |
| SDS | 0.5 % |
| Proteinase-K | 20 mg/ml |

{3.12. KSOM:}

| | | | |
|---|---|---|---|
| | | | 100 ml: |
| NaCl | No.: S-5886 | Sigma | 555 mg |
| KCl | No.: P-4505 | Sigma | 18.5 mg |
| KH₂PO₄ | No.: P-5655 | Sigma | 4.75 mg |
| MgSO4 | No.: M-7774 | Sigma | 4.95 mg |
| CaCl₂ | No.: C-7902 | Sigma | 25 mg |
| NaHCO₃ | No.: S-5761 | Sigma | 210 mg |
| Glucose | No.: G-6152 | Sigma | 3.6 mg |
| Na-Pyruvate | No.: P-4562 | Sigma | 2.2 mg |
| DL-Lactic-Acid | No.: L-1375 | Sigma | 174 µl |
| EDTA, 10 mM | No.: E-6635 | Sigma | 100 µl |
| Streptomycin | No.: S-9137 | Sigma | 5 mg |
| Penicillin | No.: P-7794 | Sigma | 6.3 mg |
| Phenol Red 0.5 % | No.: P-0290 | Sigma | 100 µl |
| L-Glutamine | No.: G-8540 | Sigma | 14.6 mg |
| MEM es. AA | No.: 11130-051 | Gibco | 1 ml |
| MEM non es. AA | No.: M-7145 | Sigma | 0.5 ml |
| BSA | No.: A-4378 | Sigma | 0.1 g |

KSOM/HEPES:

| | |
|---|---|
| add to KSOM: | |
| NaHCO₃ | 15 mM |
| HEPES pH 7.4 | 20.85 mM |

{3.13. 20 x SSC:}
3 M NaCl
0.2 M sodium citrate
{3.14. 20 x SSPE:}
3 M NaCl
0.2 M NaH₂PO₄
0.02 M EDTA
{3.15. Hybridization buffer:}

| | | |
|---|---|---|
| 7 % (w/v) SDS | No.: L-6026 | Sigma |
| 10 % (w/v) Polyethylenglykol 6000 | No.: 81255 | Sigma |
| 1.5 x SSPE (20x) | | |
| 200 µg/ml denatured herring sperm DNA | No.: D1816 | Promega |

| {3.12. Other Material} | | |
|---|---|---|
| Mineral oil | No.: M-8410 | Sigma |
| 0.1% Gelatin 100 ml, | No.: G1393 | Sigma |
| Mitomycin C | No.: M-0503 | Sigma |
| Proteinase-K | No.: 0311584401 | Roche |
| DNase | No.: D-4527 | Sigma |

### {4. Glomerular expression of hPLA2R in knockin mice of the invention}

hPLA2R/GPI mice of the invention were bred with Podo-Cre mice [6], in order to generate "Podo-Cre hPLA2R/GPI" mice according to the invention. To confirm expression of hPLA2R in podocytes of these mice, immunohistological investigations were performed on kidney tissue sections. It turned out that both the eGFP gene and the hPLA2R gene, which had been introduced into the mice via the target vector into the Rosa26 locus, were expressed in the glomerulus. Moreover, the location of the stained cells corresponds to the typical distribution of podocytes in the glomerular tuft (figure 3, arrows).

For further testing, "Podo-Cre hPLA2R/GPI" mice and "hPLA2R/GPI" mice of the invention were actively immunised with recombinant human PLA2R, and the generation of specific immune complexes was investigated. Immune complexes were only found in "Podo-Cre hPLA2R/GPI" mice along the glomerular basement membrane, but not in hPLA2R/GPI mice that do not express hPLA2R due to the lacking Cre recombinase (not shown). This also proves the excellent functionality of the invention.

### {Sequence overview}

Overview of sequences referred to in the application:

| SEQ ID NO: | Type | Annotation |
|---|---|---|
| 01 | mRNA | PLA2R1, extracellular domains |
| 02 | DNA | hPLA2R/GPI target vector |
| 03 | DNA | DANOD fw primer |
| 04 | DNA | DANOD rev primer |
| 05 | DNA | DANOD screening fw primer |
| 06 | DNA | DANOD screening-rev-Primer |
| 07 | DNA | R26 fw-primer |
| 08 | DNA | R26 rev primer |
| 09 | DNA | R26 screening fw primer |
| 10 | DNA | R26 screening rev primer |
| 11 | DNA | PLA2R/GPI genot. fw primer |
| 12 | DNA | PLA2R/GPI genot. rev primer |
| 13 | DNA | CD8 leader sequence |
| 14 | DNA | GPI anchor seqence |

### {Citation List}

### {Non Patent Literature}

[1] Beck LH Jr, Bonegio RG, Lambeau G, Beck DM, Powell DW, Cummins TD, Klein JB, Salant DJ (2009). M-type phospholipase A2 receptor as target antigen in idiopathic membranous nephropathy. N Engl J Med 361: 11-21.
[2] Ancian P, Lambeau G, Mattéi M-G, Lazdunski M (1995). The human 180-kDa receptor for secretory phospholioase A2. J Biol Chem 270: 8963-8970.
[3] Maeda Y, Kinoshita T (2011). Structural remodeling, trafficking and functions of glycosylphosphatidyl-anchored proteins. Prog Lipid Res 50: 411-424.
[4] Bannas P, Scheuplein F, Well L, Hermans-Borgmeyer I, Haag F, Koch-Nolte F (2011). Transgenic overexpression of toxin-related Ecto-ADP-Ribosyltransferase ART2.2 sensitizes T cells but not B cells to NAD-induced cell death. Mol Immunol 48: 1762-1770.
[5] Thai TH, Calado DP, CasolaS, Ansel KM, Xiao C, Xue Y, Murphy A, Frendewey D, Valenzuela D, Kutok JL, Schmidt-supprian M, Rajewski N, Yancopoulos G, Rao A, Rajewski K (2007). Regulation of the germinal center response by micro-RNA-155. Science 316: 604-608.
[6] Möller M, Sandem SK, Soofi A, Wiggins RC, Holzman LB (2003). Podocyte-specific expression of Cre-recombinase in transgenic mice. Genesis 35: 39-42
[7] Borza DB, Zhang JJ, Beck Jr LH, Meyer-Schwesinger C., Luo W. (2013). Mouse models of membranous nephropathy: the road less travelled by, Am J Clin Exp Immunol 2013;2(2):135-145
[8] Dragatsis I. and Zeitlin S. (2001). A method for the generation of conditional gene repair mutations in mice. Nucleic Acids Res. Feb 1, 2001; 29(3): e10.
[9] Michael AJ Ferguson, Taroh Kinoshita, and Gerald W Hart. In: Varki A, Cummings RD, Esko JD, et al., editors. Essentials of Glycobiology. 2nd edition. Cold Spring Harbor (NY): Cold Spring Harbor Laboratory Press; 2009, Chapter 11: Glycosylphosphatidylinositol Anchors

## Claims

1. A genetically engineered murine animal, having expressibly integrated into the genome of all its germ cells and somatic cells a nucleic acid sequence coding for a fusion protein comprising the extracellular domains of the human phospholipase A2 receptor hPLA2R, an ER targeting signal peptide and a membrane anchoring domain.

2. The genetically engineered murine animal of claim 1, wherein the membrane anchoring domain is or comprises a GPI-addition signal peptide domain, and/or wherein the ER targeting signal peptide is or comprises a human CD8 leader sequence.

3. The genetically engineered murine animal of one of claims 1 or 2, wherein the nucleic acid further encodes a reporter protein, preferably eGFP.

4. The genetically engineered murine animal of one of claims 1 to 3, being transfected with a vector comprising or having a sequence according to SEQ ID NO: 2.

5. A genetically engineered murine animal, obtainable by transfecting an embryonic stem cell of a murine animal with a vector comprising a nucleic acid encoding a fusion protein comprising the extracellular domains of hPLA2R, an ER targeting signal peptide and a membrane anchoring domain, integrating the transfected embryonic stem cell into a blastocyst of a non-transfected murine animal, crossing the chimeric animal resulting from the blastocyst with a non-transfected murine animal and selecting a transfected murine animal from the offspring.

6. The genetically engineered murine animal of claim 5, wherein the nucleic acid further encodes a reporter protein, preferably eGFP.

7. The genetically engineered murine animal of one of claims 5 or 6, wherein the embryonic stem cell is transfected with a vector comprising or having a sequence according to SEQ ID NO: 2.

8. A genetically engineered murine animal, expressing exclusively in its podocytes a fusion protein comprising the extracellular domains of the human phospholipase A2 receptor hPLA2R, an ER targeting signal peptide and a membrane anchoring domain.

9. The genetically engineered murine animal of claim 8, further expressing a recombinase under a podocyte-specific promoter.

10. The genetically engineered murine animal of claims 8 or 9, obtained by crossing a murine animal according to one of claims 1 to 4, or a murine animal according to one of claims 5 to 7, with a genetically engineered murine animal expressing a recombinase exclusively in its podocytes.

11. The genetically engineered murine animal of claim 10, wherein the recombinase is a Cre recombinase.

12. The genetically engineered murine animal of one of claims 1 to 11, wherein the murine animal is a mouse or rat, preferably a mouse.

13. Process for manufacturing a genetically engineered murine animal, comprising the steps of transfecting an embryonic stem cell of a murine animal with a vector comprising a nucleic acid encoding a fusion protein comprising the extracellular domains of the human phospholipase A2 receptor hPLA2R, an ER targeting signal peptide and a membrane anchoring domain, integrating the embryonic stem cell into a blastocyst of a non-transfected murine animal, crossing the chimeric animal resulting from the blastocyst with a non-transfected murine animal and selecting a transfected murine animal from the offspring.

14. Process for manufacturing a genetically engineered murine animal according to claim 13, further crossing the transfected murine animal produced with a genetically engineered murine animal expressing a recombinase, preferably a Cre recombinase, exclusively in its podocytes.

15. Process for manufacturing a genetically engineered murine animal according to one of claims 13 or 14, wherein the murine animal is a mouse or rat, preferably a mouse.


SEQUENCE LISTING

<110> UniversitÃ¤tsklinikum Hamburg-Eppendorf (UKE)

<120> Genetically engineered murine animal as a model for Primary
Membraneous Nephropathy

<130> PAT 1474

<160> 14

<170> BiSSAP 1.3

<210> 1
<211> 3912
<212> RNA
<213> Homo sapiens

<220>
<223> hPLA2R extracellular domains

<400> 1
gagggtgtgg cggcggcgct tacccccgag cggctcctgg agtggcagga taaaggaata 60

tttgttatcc aaagtgagag tctcaagaaa tgcattcaag caggtaaatc ggttctgacc 120

ctggagaact gcaagcaagc aaacaagcac atgctgtgga aatgggtttc aaaccatggc 180

ctctttaaca taggaggcag tggttgcctg ggcctgaatt tctccgcccc agagcagcca 240

ttaagcttat atgaatgtga ctccaccctc gtttccttac ggtggcgctg taacaggaag 300

atgatcacag gcccgctgca gtactctgtc caggtggcgc atgacaacac agtggtggcc 360

tcacggaagt atattcataa gtggatttct tatgggtcag gtggtggaga catttgtgaa 420

tatctacaca aagatttgca tacaatcaaa gggaacaccc acgggatgcc gtgtatgttt 480

cccttccagt ataaccatca gtggcatcat gaatgtaccc gtgaaggtcg ggaagatgac 540

ttactgtggt gtgccacgac aagccgttat gaaagagatg aaaagtgggg attttgccct 600

gatcccacct ctgcagaagt aggttgtgat actatttggg agaaggacct caattcacac 660

atttgctacc agttcaacct gctttcatct ctctcttgga gtgaggcaca ttcttcatgc 720

cagatgcaag gaggtacgct gttaagtatt acagatgaaa ctgaagaaaa tttcataagg 780

gagcacatga gcagtaaaac agtggaggtg tggatgggcc tcaatcagct ggatgaacac 840

gctggctggc agtggtctga tggaacgccg ctcaactatc tgaattggag cccagaggta 900

aattttgagc catttgttga agatcactgt ggaacattta gttcatttat gccaagtgcc 960

tggaggagtc gggattgtga gtccaccttg ccatatatat gtaaaaaata tctaaaccac 1020

attgatcatg aaatagttga aaaagatgcg tggaaatatt atgctaccca ctgtgagcct 1080

ggctggaatc cctacaatcg taattgctac aaacttcaga aagaagaaaa gacctggcat 1140

gaggctctgc gttcttgtca ggctgataac agtgcattaa tagacataac ctcattagca 1200

gaggtggagt ttcttgtaac cctccttgga gatgaaaatg catcagaaac atggattggt 1260

ttgagcagca ataaaattcc agtttccttt gaatggtcta atgactcttc agtcatcttt 1320

actaattggc acacacttga gccccacatt tttccaaata gaagccagct gtgtgtctca 1380

gcagagcagt ctgagggaca ctggaaagtc aaaaattgtg aagaaagact tttttacatt 1440

tgtaaaaaag caggccatgt cctctctgat gctgaatcag gatgtcaaga gggatgggag 1500

agacatggtg gattctgtta caaaattgac acagtccttc gaagctttga ccaagcttcc 1560

agcggttatt actgtcctcc tgcacttgta accattacaa acaggtttga acaggctttt 1620

attaccagtt tgatcagtag tgtggtaaaa atgaaggaca gttatttttg gatagctctt 1680

caggaccaaa atgatacggg agaatacact tggaagccag tagggcagaa acccgagccg 1740

gtgcagtaca cacactggaa cacacaccag ccgcgctaca gtggtggctg tgttgccatg 1800

cgaggaaggc atccacttgg tcgctgggaa gtgaagcact gtcggcactt taaggcaatg 1860

tccttgtgca agcagccagt tgaaaatcag gaaaaagcag agtatgaaga gagatggccc 1920

tttcacccct gctatttgga ctgggagtca gagcctggtc tggccagttg cttcaaggta 1980

tttcatagtg aaaaagttct gatgaaaaga acatggagag aagctgaagc attttgcgaa 2040

gaatttggag ctcatcttgc aagctttgcc catattgagg aagagaattt tgtgaatgag 2100

ctcttacatt caaaatttaa ttggacagaa gaaaggcagt tctggattgg atttaataaa 2160

agaaacccac tgaatgccgg ctcatgggag tggtctgata gaactcctgt tgtctcttcg 2220

tttttagaca acacttattt tggagaagat gcaagaaact gtgctgttta taaggcaaac 2280

aaaacattgc tgcccttaca ctgtggttcc aaacgtgaat ggatatgcaa aatcccaaga 2340

gatgtgaaac ccaagattcc gttctggtac cagtacgatg taccctggct cttttatcag 2400

gatgcagaat acctttttca tacctttgcc tcagaatggt tgaactttga gtttgtctgt 2460

agctggctgc acagtgatct tctcacaatt cattctgcac atgagcaaga attcatccac 2520

agcaaaataa aagcgctatc aaagtatggt gcaagttggt ggattggact tcaagaagaa 2580

agagccaatg atgaatttcg ctggagagat ggaacaccag tgatatacca gaactgggac 2640

acaggaagag aaagaactgt gaataatcag agccagagat gtggctttat ttcttctata 2700

acaggactct ggggtagtga agagtgttca gtttctatgc ctagtatctg taagcgaaaa 2760

aaggtttggc tcatagagaa aaagaaagat acaccaaaac aacatggaac gtgtcccaaa 2820

ggatggctat attttaacta taagtgcctt ctgctgaata tccccaaaga cccaagcagt 2880

tggaagaact ggacgcatgc tcaacatttc tgtgctgaag aaggggggac cctggtcgcc 2940

attgaaagtg aggtggagca agctttcatt actatgaatc tttttggcca gaccaccagt 3000

gtgtggatag gtttacaaaa tgatgattat gaaacatggc taaatggaaa gcctgtggta 3060

tattctaact ggtctccatt tgatataata aatattccaa gtcacaatac cactgaagtt 3120

cagaaacaca ttcctctctg tgccttactc tcaagtaatc ctaattttca tttcactgga 3180

aaatggtatt ttgaagactg tggaaaggaa ggctatgggt ttgtttgtga aaaaatgcaa 3240

gatacttctg gacacggtgt aaatacatct gatatgtatc caatgcccaa taccttagaa 3300

tatggaaaca gaacttacaa aataattaat gcaaatatga cttggtatgc agcaataaaa 3360

acctgcctga tgcacaaagc acaactggtc agcatcacag accagtatca ccagtccttc 3420

ctcactgttg tcctcaaccg gctaggatat gcccactgga ttggactgtt caccacagat 3480

aatggtctta attttgactg gtctgatggc accaaatctt ctttcacttt ttggaaagat 3540

gaggagtcct ccctccttgg tgactgcgtt tttgccgaca gcaacggacg ctggcatagc 3600

acagcctgcg agtcatttct gcaaggtgcc atttgtcatg tgccacctga aacaagacaa 3660

tctgaacacc cagagttgtg ctcagaaaca tctattccct ggataaaatt taaaagtaat 3720

tgctacagtt tttctacagt cctagacagt atgagttttg aggctgctca tgaattttgc 3780

aaaaaggaag gttctaatct tttaacaatc aaggatgagg ctgaaaatgc atttctccta 3840

gaagagctgt ttgcttttgg ttcttctgtc cagatggttt ggttgaatgc tcaatttgat 3900

ggtaacaatg aa 3912

<210> 2
<211> 16103
<212> DNA
<213> Artificial Sequence

<220>
<223> Targeting vector

<400> 2
ccccgcggca ggccctccga gcgtggtgga gccgttctgt gagacagccg ggtacgagtc 60

gtgacgctgg aaggggcaag cgggtggtgg gcaggaatgc ggtccgccct gcagcaaccg 120

gagggggagg gagaagggag cggaaaagtc tccaccggac gcggccatgg ctcggggggg 180

ggggggcagc ggaggacgct tccggccgac gtctcgtcgc tgattggctt ttttcctccc 240

gccgtgtgtg aaaacacaaa tggcgtgttt tggttggcgt aaggcgcctg tcagttaacg 300

gcagccggag tgcgcagccg ccggcagcct cgctctgccc actgggtggg gcgggaggta 360

ggtggggtga ggcgagctga cgtgcgggcg cggtcggcct ctggcggggc gggggagggg 420

agggagggtc agcgaaagta gctcgcgcgc gagcggccgc ccaccctccc cttcctctgg 480

gggagtcgtt ttacccgccg ccggccgggc ctcgtcgtct gattggctct cggggcccag 540

aaaactggcc cttgccattg gctcgtgttc gtgcaagttg agtccatccg ccggccagcg 600

ggggcggcga ggaggcgctc ccaggttccg gccctcccct cggccccgcg ccgcagagtc 660

tggccgcgcg cccctgcgca acgtggcagg aagcgcgcgc tgggggcggg gacgggcagt 720

agggctgagc ggctgcgggg cgggtgcaag cacgtttccg acttgagttg cctcaagagg 780

ggcgtgctga gccagacctc catcgcgcac tccggggagt ggagggaagg agcgagggct 840

cagttgggct gttttggagg caggaagcac ttgctctccc aaagtcgctc tgagttgtta 900

tcagtaaggg agctgcagtg gagtaggcgg ggagaaggcc gcacccttct ccggaggggg 960

gaggggagtg ttgcaatacc tttctgggag ttctctgctg cctcctggct tctgaggacc 1020

gccctgggcc tgggagaatc ccttgccccc tcttcccctc gtgatctgca actccagtct 1080

ttctagcatc tgtagggcgc agtagtccag ggtttccttg atgatgtcat acttatcctg 1140

tccctttttt ttccacagct cgcggttgag gacaaactct tcgcggtctt tccagtggtt 1200

aattaagtcg acattgatta ttgactagtt attaatagta atcaattacg gggtcattag 1260

ttcatagccc atatatggag ttccgcgtta cataacttac ggtaaatggc ccgcctggct 1320

gaccgcccaa cgacccccgc ccattgacgt caataatgac gtatgttccc atagtaacgc 1380

caatagggac tttccattga cgtcaatggg tggagtattt acggtaaact gcccacttgg 1440

cagtacatca agtgtatcat atgccaagta cgccccctat tgacgtcaat gacggtaaat 1500

ggcccgcctg gcattatgcc cagtacatga ccttatggga ctttcctact tggcagtaca 1560

tctacgtatt agtcatcgct attaccatgg tcgaggtgag ccccacgttc tgcttcactc 1620

tccccatctc ccccccctcc ccacccccaa ttttgtattt atttattttt taattatttt 1680

gtgcagcgat gggggcgggg gggggggggg cgcgccaggc ggggcggggc ggggcgaggg 1740

gcggggcggg gcgaggcgga gaggtgcggc ggcagccaat cagagcggcg cgctccgaaa 1800

gtttcctttt atggcgaggc ggcggcggcg gcggccctat aaaaagcgaa gcgcgcggcg 1860

ggcgggagtc gctgcgcgct gccttcgccc cgtgccccgc tccgccgccg cctcgcgccg 1920

cccgccccgg ctctgactga ccgcgttact cccacaggtg agcgggcggg acggcccttc 1980

tcctccgggc tgtaattagc gcttggttta atgacggctt gtttcttttc tgtggctgcg 2040

tgaaagcctt gaggggctcc gggagggccc tttgtgcggg gggagcggct cggggggtgc 2100

gtgcgtgtgt gtgtgcgtgg ggagcgccgc gtgcggctcc gcgctgcccg gcggctgtga 2160

gcgctgcggg cgcggcgcgg ggctttgtgc gctccgcagt gtgcgcgagg ggagcgcggc 2220

cgggggcggt gccccgcggt gcgggggggg ctgcgagggg aacaaaggct gcgtgcgggg 2280

tgtgtgcgtg ggggggtgag cagggggtgt gggcgcgtcg gtcgggctgc aaccccccct 2340

gcacccccct ccccgagttg ctgagcacgg cccggcttcg ggtgcggggc tccgtacggg 2400

gcgtggcgcg gggctcgccg tgccgggcgg ggggtggcgg caggtggggg tgccgggcgg 2460

ggcggggccg cctcgggccg gggagggctc gggggagggg cgcggcggcc cccggagcgc 2520

cggcggctgt cgaggcgcgg cgagccgcag ccattgcctt ttatggtaat cgtgcgagag 2580

ggcgcaggga cttcctttgt cccaaatctg tgcggagccg aaatctggga ggcgccgccg 2640

caccccctct agcgggcgcg gggcgaagcg gtgcggcgcc ggcaggaagg aaatgggcgg 2700

ggagggcctt cgtgcgtcgc cgcgccgccg tccccttctc cctctccagc ctcggggctg 2760

tccgcggggg gacggctgcc ttcggggggg acggggcagg gcggggttcg gcttctggcg 2820

tgtgaccggc ggctctagag cctctgctaa ccatgttcat gccttcttct ttttcctaca 2880

gctcctgggc aacgtgctgg ttattgtgct gtctcatcat tttggcaaag aattcctcga 2940

gttaattaaa taacttcgta tagcatacat tatacgaagt tatggatccg aacaaacgac 3000

ccaacacccg tgcgttttat tctgtctttt tattgccgat cccctcagaa gaactcgtca 3060

agaaggcgat agaaggcgat gcgctgcgaa tcgggagcgg cgataccgta aagcacgagg 3120

aagcggtcag cccattcgcc gccaagctct tcagcaatat cacgggtagc caacgctatg 3180

tcctgatagc ggtccgccac acccagccgg ccacagtcga tgaatccaga aaagcggcca 3240

ttttccacca tgatattcgg caagcaggca tcgccatggg tcacgacgag atcctcgccg 3300

tcgggcatgc gcgccttgag cctggcgaac agttcggctg gcgcgagccc ctgatgctct 3360

tcgtccagat catcctgatc gacaagaccg gcttccatcc gagtacgtgc tcgctcgatg 3420

cgatgtttcg cttggtggtc gaatgggcag gtagccggat caagcgtatg cagccgccgc 3480

attgcatcag ccatgatgga tactttctcg gcaggagcaa ggtgagatga caggagatcc 3540

tgccccggca cttcgcccaa tagcagccag tcccttcccg cttcagtgac aacgtcgagc 3600

acagctgcgc aaggaacgcc cgtcgtggcc agccacgata gccgcgctgc ctcgtcctgc 3660

agttcattca gggcaccgga caggtcggtc ttgacaaaaa gaaccgggcg cccctgcgct 3720

gacagccgga acacggcggc atcagagcag ccgattgtct gttgtgccca gtcatagccg 3780

aatagcctct ccacccaagc ggccggagaa cctgcgtgca atccatcttg ttcaatggcc 3840

gatcccatat tggctgcagg gtcgctcggt gttcgaggcc acacgcgtca ccttaatatg 3900

cgaagtggac ctgggaccgc gccgccccga ctgcatctgc gtgttcgaat tcgccaatga 3960

caagacgctg ggcggggttt gctcgacatt gggtggaaac attccaggcc tgggtggaga 4020

ggctttttgc ttcctcttgc aaaaccacac tgctcgacat tgggtggaaa cattccaggc 4080

ctgggtggag aggctttttg cttcctcttg caaaaccaca ctgctcgact agtgattaaa 4140

gtcgactcgg ggacaccaaa tatggcgatc tcggcctttt cgtttcttgg agctgggaca 4200

tgtttgccat cgatccatct accaccagaa cggccgttag atctgctgcc accgttgttt 4260

ccaccgaaga aaccaccgtt gccgtaacca ccacgacggt tgttgctaaa gaagctgcca 4320

ccgccacggc caccgttgta gccgccgttg ttgttattgt agttgctcat gttatttctg 4380

gcacttcttg gttttcctct taagtgagga ggaacataac cattctcgtt gttgtcgttg 4440

atgcttaaat tttgcacttg ttcgctcagt tcagccataa tatgaaatgc ttttcttgtt 4500

gttcttacgg aataccactt gccacctatc accacaacta actttttccc gttcctccat 4560

ctcttttata ttttttttct cgagggatct ttgtgaagga accttacttc tgtggtgtga 4620

cataattgga caaactacct acagagattt aaagctctaa ggtaaatata aaatttttaa 4680

gtgtataatg tgttaaacta ctgattctaa ttgtttgtgt attttagatt ccaacctatg 4740

gaactgatga atgggagcag tggtggaatg cctttaatga ggaaaacctg ttttgctcag 4800

aagaaatgcc atctagtgat gatgaggcta ctgctgactc tcaacattct actcctccaa 4860

aaaagaagag aaaggtagaa gaccccaagg actttccttc agaattgcta agttttttga 4920

gtcatgctgt gtttagtaat agaactcttg cttgctttgc tatttacacc acaaaggaaa 4980

aagctgcact gctatacaag aaaattatgg aaaaatattc tgtaaccttt ataagtaggc 5040

ataacagtta taatcataac atactgtttt ttcttactcc acacaggcat agagtgtctg 5100

ctattaataa ctatgctcaa aaattgtgta cctttagctt tttaatttgt aaaggggtta 5160

ataaggaata tttgatgtat agtgccttga ctagagatca taatcagcca taccacattt 5220

gtagaggttt tacttgcttt aaaaaacctc ccacacctcc ccctgaacct gaaacataaa 5280

atgaatgcaa ttgttgttgt taacttgttt attgcagctt ataatggtta caaataaagc 5340

aatagcatca caaatttcac aaataaagca tttttttcac tgcattctag ttgtggtttg 5400

tccaaactca tcaatgtatc ttatcatgtc tggatctgac atggtaagta agcttataac 5460

ttcgtatagc atacattata cgaagttatg ggcgcgccgc ggccgcgatc aattcggtac 5520

cgaagttcct attccgaagt tcctattctc tagaaagtat aggaacttcc tcgagggtac 5580

cccaattccg cccccccccc ccccctaacg ttactggccg aagccgcttg gaataaggcc 5640

ggtgtgcgtt tgtctatatg ttattttcca ccatattgcc gtcttttggc aatgtgaggg 5700

cccggaaacc tggccctgtc ttcttgacga gcattcctag gggtctttcc cctctcgcca 5760

aaggaatgca aggtctgttg aatgtcgtga aggaagcagt tcctctggaa gcttcttgaa 5820

gacaaacaac gtctgtagcg accctttgca ggcagcggaa ccccccacct ggcgacaggt 5880

gcctctgcgg ccaaaagcca cgtgtataag atacacctgc aaaggcggca caaccccagt 5940

gccacgttgt gagttggata gttgtggaaa gagtcaaatg gctctcctca agcgtattca 6000

acaaggggct gaaggatgcc cagaaggtac cccattgtat gggatctgat ctggggcctc 6060

ggtgcacatg ctttacatgt gtttagtcga ggttaaaaaa cgtctaggcc ccccgaacca 6120

cggggacgtg gttttccttt gaaaaacacg atgataatat ggccacaacc atggtgagca 6180

agggcgagga gctgttcacc ggggtggtgc ccatcctggt cgagctggac ggcgacgtaa 6240

acggccacaa gttcagcgtg tccggcgagg gcgagggcga tgccacctac ggcaagctga 6300

ccctgaagtt catctgcacc accggcaagc tgcccgtgcc ctggcccacc ctcgtgacca 6360

ccctgaccta cggcgtgcag tgcttcagcc gctaccccga ccacatgaag cagcacgact 6420

tcttcaagtc cgccatgccc gaaggctacg tccaggagcg caccatcttc ttcaaggacg 6480

acggcaacta caagacccgc gccgaggtga agttcgaggg cgacaccctg gtgaaccgca 6540

tcgagctgaa gggcatcgac ttcaaggagg acggcaacat cctggggcac aagctggagt 6600

acaactacaa cagccacaac gtctatatca tggccgacaa gcagaagaac ggcatcaagg 6660

tgaacttcaa gatccgccac aacatcgagg acggcagcgt gcagctcgcc gaccactacc 6720

agcagaacac ccccatcggc gacggccccg tgctgctgcc cgacaaccac tacctgagca 6780

cccagtccgc cctgagcaaa gaccccaacg agaagcgcga tcacatggtc ctgctggagt 6840

tcgtgaccgc cgccgggatc actctcggca tggacgagct gtacaagtaa tccgggatcc 6900

ggagagctcc caacgaagtt cctattccga agttcctatt ctctagaaag tataggaact 6960

tcctcgaggt tggatgcagc ccgggggatc cactagttct agagcggccg atcagcctcg 7020

actgtgcctt ctagttgcca gccatctgtt gtttgcccct cccccgtgcc ttccttgacc 7080

ctggaaggtg ccactcccac tgtcctttcc taataaaatg aggaaattgc atcgcattgt 7140

ctgagtaggt gtcattctat tctggggggt ggggtggggc aggacagcaa gggggaggat 7200

tgggaagaca atagcaggca tgctggggat gcggtgggct ctatggcttc tgaggcggaa 7260

agaaccagct ggggctcgag atccactagt tctagcctcg aggctagagc ggccgccgcg 7320

gatatcgaat tcgctagaag atgggcggga gtcttctggg caggcttaaa ggctaacctg 7380

gtgtgtgggc gttgtcctgc aggggaattg aacaggtgta aaattggagg gacaagactt 7440

cccacagatt ttcggttttg tcgggaagtt ttttaatagg ggcaaatagg aaaatggagg 7500

ataggagtca tctggggttt atgcagcaaa actacaggta tattgcttgt atccgcctcg 7560

gagatttcca tgaggagata aagacatgtc acccgagttt atactctcct gcttagatcc 7620

tactacagta tgaaatacag tgtgcgaggt agactatgta agcagattta atcattttaa 7680

agagcccagt acttcatatc catttctccc gctccttctg cagccttatc aaaaggtatt 7740

tagaacactc attttagccc cattttcatt tattatactg gcttatccaa cccctagaca 7800

gagcattggc attttccctt tcctgatctt agaagtctga tgactcatga aaccagacag 7860

attagttaca tacaccacaa atcgaggctg tagctggggc ctcaacactg cagttctttt 7920

ataactcctt agtacacttt ttgttgatcc tttgccttga tccttaattt tcagtgtcta 7980

tcacctctcc cgtcaggtgg tgttccacat ttgggcctat tctcagtcca gggagtttta 8040

caacaataga tgtattgaga atccaaccta aagcttaact ttccactccc atgaatgcct 8100

ctctcctttt tctccattat aactgagcta taccattaat ggtttcaggt ggatgtctcc 8160

tcccccaata tacctgatgt atctacatat tgccaggctg atattttaag acataaaggt 8220

atatttcatt attgagccac atggtattga ttactgctac taaaattttg tcattgtaca 8280

catctgtaaa aggtggttcc ttttggaatg caaagttcag gtgtttgttg tctttcctga 8340

cctaaggtct tgtgagcttg tattttttct atttaagcag tgctttctct tggactggct 8400

tgactcatgg cattctacac gttattgctg gtctaaatgt gattttgcca agcttcttca 8460

ggacctataa ttttgcttga cttgtagcca aacacaagta aaatgattaa gcaacaaatg 8520

tatttgtgaa gcttggtttt taggttgttg tgttgtgtgt gcttgtgctc tataataata 8580

ctatccaggg gctggagagg tggctcggag ttcaagagca cagactgctc ttccagaagt 8640

cctgagttca attcccagca accacatggt ggctcacaac catctgtaat gggatctgat 8700

gccctcttct ggtgtgtctg aagaccacaa gtgtattcac attaaataaa taatcctcct 8760

tcttcttctt tttttttttt taaagagaat ctgtctccag tagaattact gaagtaatga 8820

aatactttgt gtttgttcca atatggagcc aataatcaaa tactcttagc actggaaatg 8880

taccaaggaa ctattttatt taagtgactg tggacagagg agccataact gcagacttgt 8940

gggatacaga agaccaatgc agacttaatg tcttttctct tacactaagc aataaagaaa 9000

taaaaattga acttctagta tcctatttgt taaactgcta gctttactaa cttttgtgct 9060

tcatctatac aaagctgaaa gctaagtctg cagccattac taaacatgaa agcaagtaat 9120

gataattttg gatttcaaaa atgtagggcc agagtttagc cagccagtgg tggtgcttgc 9180

ctttatgcct taatcccagc actctggagg cagagacagg cagatctctg agtttgagcc 9240

cagcctggtc tacacatcaa gttctatcta ggatagccag gaatacacac agaaaccctg 9300

ttggggaggg gggctctgag atttcataaa attataattg aagcattccc taatgagcca 9360

ctatggatgt ggctaaatcc gtctaccttt ctgatgagat ttgggtatta ttttttctgt 9420

ctctgctgtt ggttgggtct tttgacactg tgggctttct taaagcctcc ttccctgcca 9480

tgtggtctct tgtttgctac taacttccca tggcttaaat ggcatggctt tttgccttct 9540

aagggcagct gctgagtttg cagcctgatt tccagggtgg ggttgggaaa tctttcaaac 9600

actaaaattg tcctttaatt tttttttaaa aaatgggtta tataataaac ctcataaaat 9660

agttatgagg agtgaggtgg actaatatta atgagtccct cccctataaa agagctatta 9720

aggctttttg tcttatacta actttttttt taaatgtggt atctttagaa ccaagggtct 9780

tagagtttta gtatacagaa actgttgcat cgcttaatca gattttctag tttcaaatcc 9840

agagaatcca aattcttcac agccaaagtc aaattaagaa tttctgactt taatgttatt 9900

tgctactgtg aatataaaat gatagctttt cctgaggcag ggtctcacta tgtatctctg 9960

cctgatctgc aacaagatat gtagactaaa gttctgcctg cttttgtctc ctgaatacta 10020

aggttaaaat gtagtaatac ttttggaact tgcaggtcag attcttttat aggggacaca 10080

ctaagggagc ttgggtgata gttggtaaat gtgtttaagt gatgaaaact tgaattatta 10140

tcaccgcaac ctacttttta aaaaaaaaag ccaggcctgt tagagcatgc taagggatcc 10200

ctaggacttg ctgagcacac aagagtagta cttggcaggc tcctggtgag agcatatttc 10260
aaaaaacaag gcagacaacc aagaaactac agtaaggtta cctgtcttta accatctgca 10320
tatacacagg gatattaaaa tattccaaat aatatttcat tcaagttttc ccccatcaaa 10380
ttgggacatg gatttctccg gtgaataggc agagttggaa actaaacaaa tgttggtttt 10440
gtgatttgtg aaattgtttt caagtgatag ttaaagccca tgagatacag aacaaagctg 10500
ctatttcgag gtctcttggt tatactcaga agcacttctt tgggtttccc tgcactatcc 10560
tgatcatgtg ctaggcctcc ttaggctgat tgttgttcaa ataacttaag tttcctgtca 10620
ggtgatgtca tatgatttca tatatcaagg caaaacatgt tatatatgtt aaacatttga 10680
cttaatgtga aagttaggtc tttgtgggtt ttgattttaa tttcaaaacc tgagctaaat 10740
aagtcatttt acatgtctta catttggtga attgtatatt gtggtttgca ggcaagactc 10800
tctgacctag taaccctcct atagagcact ttgctgggtc acaagtctag gagtcaagca 10860
tttcaccttg aagttgagac gttttgttag tgtatactag ttatatgttg gaggacatgt 10920
ttatccagaa gatattcagg actatttttg actgggctaa ggaattgatt ctgattagca 10980
ctgttagtga gcattgagtg gcctttaggc ttgaattgga gtcacttgta tatctcaaat 11040
aatgctggcc ttttttaaaa gcccttgttc tttatcaccc tgttttctac ataatttttg 11100
ttcaaagaaa tacttgtttg gatctccttt tgacaacaat agcatgtttt caagccatat 11160
tttttttcct tttttttttt ttttttggtt tttcgagaca gggtttctct gtatagccct 11220
ggctgtcctg gaactcactt tgtagaccag gctggcctcg aactcagaaa tccgcctgcc 11280
tctgcctcct gagtgccggg attaaaggcg tgcaccacca cgcctggcta agttggatat 11340
tttgtatata actataacca atactaactc cactgggtgg atttttaatt cagtcagtag 11400
tcttaagtgg tctttattgg cccttattaa aatctactgt tcactctaac agaggctgtt 11460
ggactagtgg actaagcaac ttcctacgga tatactagca gataagggtc agggatagaa 11520
actagtctag cgttttgtat acctaccagc ttatactacc ttgttctgat agaaatattt 11580
aggacatcta gcttatcgat ccgtcgacgg tatcgataag cttgatatcg aattctaccg 11640
ggtaggggag gcgcttttcc aaggcagtct gagcatgcgc ttagcagccc cgctggcact 11700
tggcgctaca caagtggcct tggcctcgca cacattccac atccaccggt aggcgccaac 11760
cggctccgtt ctttggtggc cccttcgcgc caccttctct cctcccctag tcaggaagtt 11820
cccccccgcc ccgcagctcg cgtcgtagga cgtgacaaat ggaagtagca cgtctcacta 11880
gtctcgtcag atggacagca ccgctgagca atggaagcgg gtaggccttt ggggcagcgg 11940
ccaatagcag ctttgctcct tcgctttctg ggctcagagg ctgggaaggg gtgggtccgg 12000
gggcgggctc aggggcgggc tcaggggcgg ggcgggcgcc cgaaggtcct ccggaggccc 12060
ggcattctgc acgcttcaaa agcgcacgtc tgccgcgctg ttctcctctt cctcatctcc 12120

gggcctttcg acctgcaggt cctcgccatg gatcctgatg atgttgttat tcttctaatc 12180

ttttgtatgg aaaacttttc ttcgtaccac gggactaaac ctggttatgt agattccatt 12240

caaaaaggta tacaaaagcc aaaatctggt acacaaggaa attatgacga tgattggaaa 12300

gggttttata gtaccgacaa taaatacgac gctgcgggat actctgtaga taatgaaaac 12360

ccgctctctg gaaaagctgg aggcgtggtc aaagtgacgt atccaggact gacgaaggtt 12420

ctcgcactaa aagtggataa tgccgaaact attaagaaag agttaggttt aagtctcact 12480

gaaccgttga tggagcaagt cggaacggaa gagtttatca aaaggttcgg tgatggtgct 12540

tcgcgtgtag tgctcagcct tcccttcgct gaggggagtt ctagcgttga atatattaat 12600

aactgggaac aggcgaaagc gttaagcgta gaacttgaga ttaattttga aacccgtgga 12660

aaacgtggcc aagatgcgat gtatgagtat atggctcaag cctgtgcagg aaatcgtgtc 12720

aggcgatctc tttgtgaagg aaccttactt ctgtggtgtg acataattgg acaaactacc 12780

tacagagatt taaagctcta aggtaaatat aaaattttta agtgtataat gtgttaaact 12840

actgattcta attgtttgtg tattttagat tccaacctat ggaactgatg aatgggagca 12900

gtggtggaat gcagatccta gagctcgctg atcagcctcg actgtgcctt ctagttgcca 12960

gccatctgtt gtttgcccct cccccgtgcc ttccttgacc ctggaaggtg ccactcccac 13020

tgtcctttcc taataaaatg aggaaattgc atcgcattgt ctgagtaggt gtcattctat 13080

tctggggggt ggggtggggc aggacagcaa gggggaggat tgggaagaca atagcaggca 13140

tgctggggat gcggtgggct ctatggcttc tgaggcggaa agaaccagct ggggctcgac 13200

ctcgaggggg ggcccgcggc cgccctgcag ggcgatcgct acgtacagct tttgttccct 13260

ttagtgaggg ttaattgcgc gcttggcgta atcatggtca tagctgtttc ctgtgtgaaa 13320

ttgttatccg ctcacaattc cacacaacat acgagccgga agcataaagt gtaaagcctg 13380

gggtgcctaa tgagtgagct aactcacatt aattgcgttg cgctcactgc ccgctttcca 13440

gtcgggaaac ctgtcgtgcc agctgcatta atgaatcggc caacgcgcgg ggagaggcgg 13500

tttgcgtatt gggcgctctt ccgcttcctc gctcactgac tcgctgcgct cggtcgttcg 13560

gctgcggcga gcggtatcag ctcactcaaa ggcggtaata cggttatcca cagaatcagg 13620

ggataacgca ggaaagaaca tgtgagcaaa aggccagcaa aaggccagga accgtaaaaa 13680

ggccgcgttg ctggcgtttt tccataggct ccgcccccct gacgagcatc acaaaaatcg 13740

acgctcaagt cagaggtggc gaaacccgac aggactataa agataccagg cgtttccccc 13800

tggaagctcc ctcgtgcgct ctcctgttcc gaccctgccg cttaccggat acctgtccgc 13860

ctttctccct tcgggaagcg tggcgctttc tcatagctca cgctgtaggt atctcagttc 13920

ggtgtaggtc gttcgctcca agctgggctg tgtgcacgaa ccccccgttc agcccgaccg 13980

ctgcgcctta tccggtaact atcgtcttga gtccaacccg gtaagacacg acttatcgcc 14040

actggcagca gccactggta acaggattag cagagcgagg tatgtaggcg gtgctacaga 14100

gttcttgaag tggtggccta actacggcta cactagaagg acagtatttg gtatctgcgc 14160

tctgctgaag ccagttacct tcggaaaaag agttggtagc tcttgatccg gcaaacaaac 14220

caccgctggt agcggtggtt tttttgtttg caagcagcag attacgcgca gaaaaaaagg 14280

atctcaagaa gatcctttga tcttttctac ggggtctgac gctcagtgga acgaaaactc 14340

acgttaaggg attttggtca tgagattatc aaaaaggatc ttcacctaga tccttttaaa 14400

ttaaaaatga agttttaaat caatctaaag tatatatgag taaacttggt ctgacagtta 14460

ccaatgctta atcagtgagg cacctatctc agcgatctgt ctatttcgtt catccatagt 14520

tgcctgactc cccgtcgtgt agataactac gatacgggag ggcttaccat ctggccccag 14580

tgctgcaatg ataccgcgag acccacgctc accggctcca gatttatcag caataaacca 14640

gccagccgga agggccgagc gcagaagtgg tcctgcaact ttatccgcct ccatccagtc 14700

tattaattgt tgccgggaag ctagagtaag tagttcgcca gttaatagtt tgcgcaacgt 14760

tgttgccatt gctacaggca tcgtggtgtc acgctcgtcg tttggtatgg cttcattcag 14820

ctccggttcc caacgatcaa ggcgagttac atgatccccc atgttgtgca aaaaagcggt 14880

tagctccttc ggtcctccga tcgttgtcag aagtaagttg gccgcagtgt tatcactcat 14940

ggttatggca gcactgcata attctcttac tgtcatgcca tccgtaagat gcttttctgt 15000

gactggtgag tactcaacca agtcattctg agaatagtgt atgcggcgac cgagttgctc 15060

ttgcccggcg tcaatacggg ataataccgc gccacatagc agaactttaa aagtgctcat 15120

cattggaaaa cgttcttcgg ggcgaaaact ctcaaggatc ttaccgctgt tgagatccag 15180

ttcgatgtaa cccactcgtg cacccaactg atcttcagca tcttttactt tcaccagcgt 15240

ttctgggtga gcaaaaacag gaaggcaaaa tgccgcaaaa aagggaataa gggcgacacg 15300

gaaatgttga atactcatac tcttcctttt tcaatattat tgaagcattt atcagggtta 15360

ttgtctcatg agcggataca tatttgaatg tatttagaaa aataaacaaa taggggttcc 15420

gcgcacattt ccccgaaaag tgccacctaa attgtaagcg ttaatatttt gttaaaattc 15480

gcgttaaatt tttgttaaat cagctcattt tttaaccaat aggccgaaat cggcaaaatc 15540

ccttataaat caaaagaata gaccgagata gggttgagtg ttgttccagt ttggaacaag 15600

agtccactat taaagaacgt ggactccaac gtcaaagggc gaaaaaccgt ctatcagggc 15660

gatggcccac tacgtgaacc atcaccctaa tcaagttttt tggggtcgag gtgccgtaaa 15720

gcactaaatc ggaaccctaa agggagcccc cgatttagag cttgacgggg aaagccggcg 15780

aacgtggcga gaaaggaagg gaagaaagcg aaaggagcgg gcgctagggc gctggcaagt 15840

gtagcggtca cgctgcgcgt aaccaccaca cccgccgcgc ttaatgcgcc gctacagggc 15900

gcgtcccatt cgccattcag gctgcgcaac tgttgggaag ggcgatcggt gcgggcctct     15960

tcgctattac gccagctggc gaaaggggga tgtgctgcaa ggcgattaag ttgggtaacg     16020

ccagggtttt cccagtcacg acgttgtaaa acgacggcca gtgagcgcgc gtaatacgac     16080

tcactatagg gcgaattgga gct                                             16103


<210> 3
<211> 33
<212> DNA
<213> Artificial Sequence

<220>
<223> DANOD Fw primer

<400> 3
ttttttagat ctgagggtgt ggcggcggcg ctt                                    33


<210> 4
<211> 38
<212> DNA
<213> Artificial Sequence

<220>
<223> DANOD Rev primer

<400> 4
ttttttcaat tgttcttact gttaccatca aattgagc                               38


<210> 5
<211> 21
<212> DNA
<213> Artificial Sequence

<220>
<223> DANOD Screening Fw primer

<400> 5
cttgctcctg ccgctagcct t                                                21


<210> 6
<211> 23
<212> DNA
<213> Artificial Sequence

<220>
<223> DANOD Screening Rev primer

<400> 6
caaggaacag ggatacacag ctt                                              23


<210> 7
<211> 41
<212> DNA
<213> Artificial Sequence

<220>
<223> R26 Fw primer

<400> 7
aaaaaggcgc gccttagcga tggccttacc agtgaccgcc t                41


<210> 8
<211> 37
<212> DNA
<213> Artificial Sequence

<220>
<223> R26 Rev primer

<400> 8
aaaaaaggcg cgccctacgg ctcagcaaga caaagca                     37


<210> 9
<211> 25
<212> DNA
<213> Artificial Sequence

<220>
<223> R26 Screening Fw primer

<400> 9
aatagaactc ttgcttgctt tgcta                                  25


<210> 10
<211> 21
<212> DNA
<213> Artificial Sequence

<220>
<223> R26 Screening Rev primer

<400> 10
tcttcaagaa gcttccagag g                                      21


<210> 11
<211> 21
<212> DNA
<213> Artificial Sequence

<220>
<223> PLA2R/GPI genot. Fw Primer

<400> 11
ttaacaatca aggatgaggc t                                      21


<210> 12
<211> 21
<212> DNA
<213> Artificial Sequence

<220>
<223> PLA2R/GPI genot. Rev primer

<400> 12
tcttcaagaa gcttccagag g                                                21

<210> 13
<211> 63
<212> DNA
<213> Homo sapiens

<220>
<223> CD8 leader

<400> 13
atggccttac cagtgaccgc cttgctcctg ccgctagcct tgctgctcca cgccgccagg      60

ccg                                                                    63

<210> 14
<211> 90
<212> DNA
<213> Artificial Sequence

<220>
<223> GPI-addition signal

<400> 14
agcgcaggag ccagagaaag ctgtgtatcc ctgttccttg tggttctccc cagtcttctg      60

gtccagctgc tttgtcttgc tgagccgtag                                       90